# EUROPEAN PATENT SPECIFICATION

(11) **EP 1 755 634 B1**
(45) Date of publication and mention of the grant of the patent: **08.10.2008**
(21) Application number: 05729655.0
(22) Date of filing: 08.04.2005
(51) Int. Cl.: A61K 38/06, A61K 38/07, A61K 38/08, A61P 13/12

(54) **OLIGOPEPTIDES FOR REDUCING ELEVATED BLOOD UREA CONCENTRATION**
OLIGOPEPTIDE ZUR VERRINGERUNG ERHÖHTER BLUTHARNSTOFFKONZENTRATION
OLIGOPEPTIDES PERMETTANT DE REDUIRE UNE CONCENTRATION ELEVEE D'UREE SANGUINE

(30) Priority: 08.04.2004 US 821256
(43) Date of publication of application: 28.02.2007
(73) Proprietor: Biotempt B.V., 7958 NZ Koekange (NL)
(72) Inventor: KHAN, Nisar, Ahmed, 3039 JL Rotterdam (NL); BENNER, Robbert, NL-2992 SH Barendrecht (NL)
(74) Representative: van Loon, C.J.J.
(86) International application number: PCT/EP2005/003707
(87) International publication number: WO 2005/097163

(56) References cited:
- WO-A-01/68113
- US-A1- 2003 220 258
- US-A1- 2003 220 261
- US-A1- 2004 013 661

### SEQUENCE LISTING

<110> Khan, Nisar A
   Benner, Robbert
<120> Compositions Capable of Reducing Elevated Blood Urea Concentration
<130> 3077-6420PC
<160> 4
<170> PatentIn version 3.2
<210> 1
   <211> 4
   <212> PRT
   <213> Artificial Sequence
<220>
   <223> Synthesized Peptide
<400> 1
   Met Thr Arg Val
   1
<210> 2
   <211> 4
   <212> PRT
   <213> Artificial Sequence
<220>
   <223> Synthesized Peptide
<400> 2
   Ala Gln Gly Val
   1
<210> 3
   <211> 4
   <212> PRT
   <213> Artificial Sequence
<220>
   <223> Synthesized Peptide
<400> 3
   Leu Gln Gly Val
   1
<210> 4
   <211> 4
   <212> PRT
   <213> Artificial Sequence
<220>
   <223> Synthesized Peptide
<400> 4
   Leu Ala Gly Val
   1

## Description

### TECHNICAL FIELD

The invention relates generally to biotechnology, and more specifically to compositions having immunoregulatory activity, which compounds include particular oligopeptides derived from human chorionic gonadotropin (hCG).

### BACKGROUND

U.S. Patent 5,380,668 to Herron (Jan. 10, 1995), the contents of the entirety of which are incorporated by this reference, discloses, among other things, various compounds having the antigenic binding activity of hCG. The oligopeptides disclosed therein are disclosed generally for use in diagnostic methods.

Various patents and patent applications to Gallo et al. (e.g., U.S. Patent 5,677,275 (corresponding to WO 96/04008 A1), U.S. Patent 5,877,148 (also corresponding to WO 96/04008 A1), WO 97/49721 A1, U.S. Patent 6,319,504 (corresponding to WO 97/49373), U.S. Patent Application 2003/0049273 A1 (also corresponding to WO 97/49373), U.S. Patent 5,968,513 (corresponding to WO 97/49418), U.S. Patent 5,997,871 (corresponding to WO 97/49432), U.S. Patent 6,620,416, U.S. Patent 6,596,688, WO 01/11048 A2, WO 01/10907 A2., and U.S. Patent 6,583,109) relate to various oligopeptides and their use in, among other things, "inhibiting HIV infection," "treating or preventing HIV infection," "treating or preventing cancer," "treating or preventing a condition characterized by loss of body cell mass," "treating or preventing a condition associated with pathological angiogenesis," "treating or preventing hematopoietic deficiency," "ex vivo gene therapy," "expanding blood cells in vitro," and/or "providing blood cells to a subject."

### DISCLOSURE OF THE INVENTION

As we described in PCT International Publication No. WO 03/029292 A2 (published April 10, 2003), PCT International Publication No. WO 01/72831 A2 (published October 4, 2001), and U.S. Patent Application Publications 20020064501 A1 (published May 30, 2002), 20030119720 A1 (published June 26, 2003), 20030113733 A1 (published June 19, 2003), and 20030166556 A1 (published September 4, 2003), the contents of all of which are incorporated by this reference, compositions containing some of the oligopeptides described herein have immunoregulatory activity useful in, for example, the treatment of sepsis and other disease states and conditions.

The invention includes a method of reducing blood urea nitrogen (BUN) concentration (herein also called urea concentration) in a subject's serum. Such a method comprises administering to the subject (e.g., a mammal such as a human) a composition comprising an oligopeptide (or oligopeptides) having activity in reducing urea concentration in the subject's serum as determined by a mouse renal reperfusion test, wherein the oligopeptide has the sequence AQGV (SEQ ID NO:2) or MTRV (SEQ ID NO:1).

In the case where the composition only includes the oligopeptide AQGV (SEQ ID NO:2), the composition may be administered orally. The oligopeptide will preferably be of synthetic origin (e.g., produced by a Merrifield synthesis). When the composition is administered to the subject parenterally, the composition will typically consist essentially of oligopeptide and PBS (e.g., in an amount of from about 0.25 to about 10 mg/kg body mass of the subject).

The invention is thought to be useful for instances, when, for example, the subject is undergoing acute renal failure, especially when the subject is undergoing persistent oliguria, is not producing more than ½ ml urine per hour per kilogram body mass of the subject, and/or has a serum potassium level greater than 6.5 mmol per liter serum.

In one preferred embodiment, the invention involves administering a purified, synthetic or isolated peptide consisting of AQGV (SEQ ID NO:2), or an acid addition salt thereof.

The invention also provides use of a composition according to the invention for the preparation of a pharmaceutical composition or medicament for the treatment of a disorder such as acute renal failure.

### BRIEF DESCRIPTION OF THE FIGURE

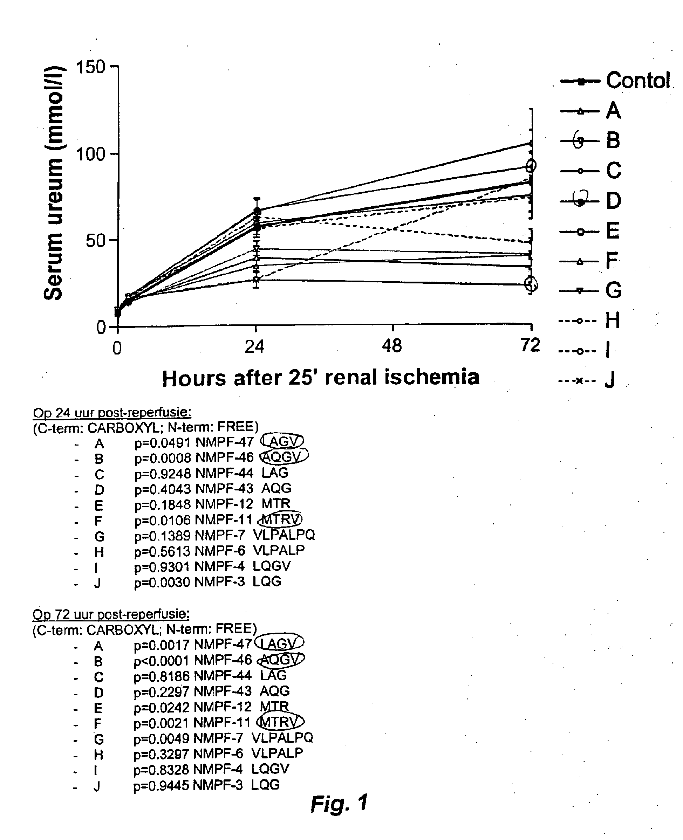

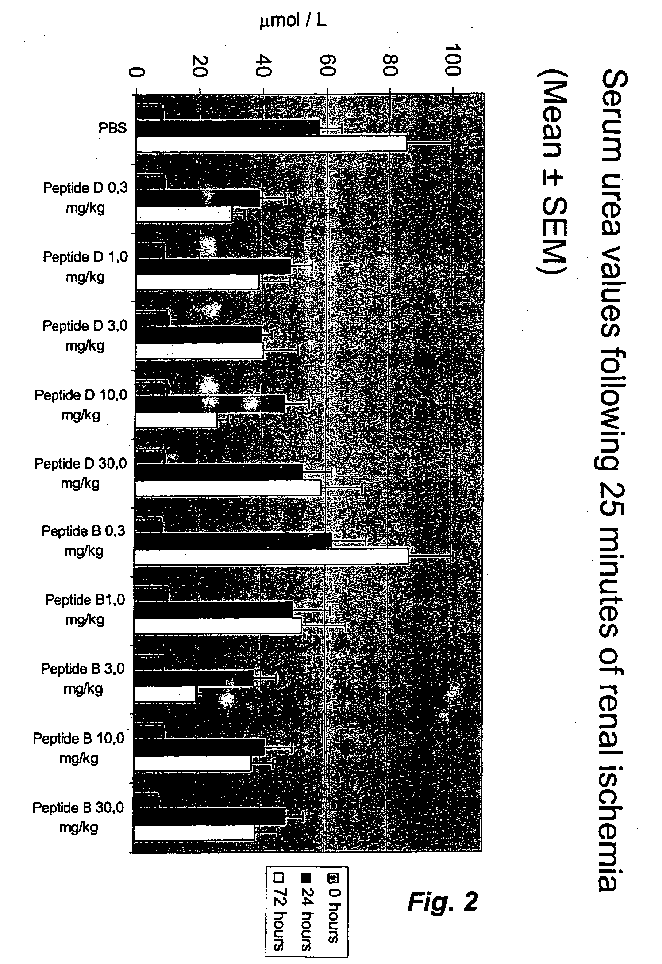

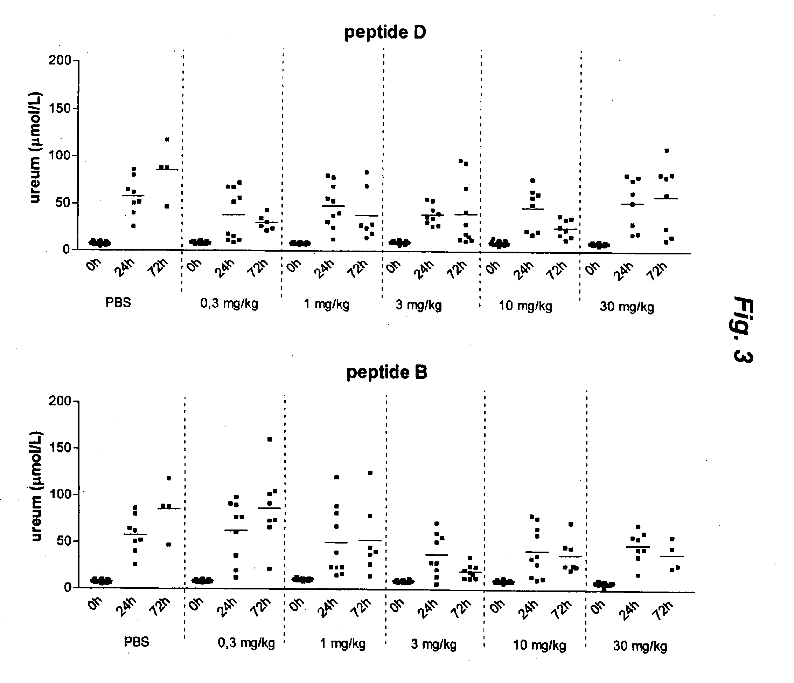

FIG. 1 graphically depicts the results of the examples; shown are the BUN (urea) values at the various points in time after treatment with peptides A to F or without treatment (control).

### DETAILED DESCRIPTION OF THE INVENTION

As used herein, a "purified, synthetic or isolated" peptide is one that has been purified from a natural or biotechnological source, or, more preferably, is synthesized as described herein.

"Composition," as used herein, refers to chemical compounds that contain or consist of the oligopeptide. The oligopeptide is preferably isolated before inclusion within the composition.

For instance, the previously described preferred compound could, in one embodiment be;
NT A Q G V CT
wherein NT at the N-terminus is selected from the group of H--, CH3--, an acyl group, or a general protective group; and CT at the C-terminus is selected from the group of small (e.g., 1 to 5 amino acids) peptides, --OH, --OR1, --NH2, -NHR1, --NR1 R2, or --N(CH2)1-6 NR1 R2, wherein R1 and R2, when present, are independently selected from H, alkyl, aryl, (ar)alkyl, and wherein R1 and R2 can be cyclically bonded to one another.

"Alkyl" as used herein, is preferably a saturated branched or unbranched hydrocarbon having one to six carbon atoms, e.g. methyl, ethyl, and isopentyl.

"Aryl" as used herein, is an aromatic hydrocarbon group, preferably having 6 to 10 carbon atoms, such as phenyl or naphthyl.

"(Ar)alkyl" as used herein, is an arene group (having both aliphatic and aromatic portions), preferably having 7 to 13 carbon atoms such as benzyl, ethylbenzyl, n-propylbenzyl, and isobutylbenzyl.

"Oligopeptide" as used herein, are peptides having from 3 to 12 amino acids joined together by peptide bonds. Equivalent to oligopeptides are compounds having the same or equivalent sidechains as the particular amino acids used in an oligopeptide, and arranged sequentially in the same order as the peptides, but joined together by non-peptide bonds, e.g., by isosteric linkages such as the keto isostere, hydroxy isostere, diketo isostere, or the keto-difluoromethylene isostere.

"Composition" also includes, for example, an acceptable salt of the oligopeptide or a labeled oligopeptide. As used herein, "acceptable salt" refers to salts that retain the desired activity of the oligopeptide or equivalent compound, but preferably do not detrimentally affect the activity of the oligopeptide or other component of a system in which uses the oligopeptide. Examples of such salts are acid addition salts formed with inorganic acids, for example, hydrochloric acid, hydrobromic acid sulfuric acid, phosphoric acid, nitric acid, and the like. Salts may also be formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, and the like. Salts may be formed with polyvalent metal cations such as zinc, calcium, bismuth, barium, magnesium, aluminum, copper, cobalt, nickel and the like or with an organic cation formed from N,N'-dibenzylethylenediamine or ethylenediaminc, or combinations thereof (e.g., a zinc tannate salt).

The invention also provides use of an oligopeptide having activity in reducing urea concentration in a subject's serum as determined by a mouse renal reperfusion test, the oligopeptide having the sequence AQGV or MTRV (SEQ ID NO: 1), in the production of a pharmaceutical composition for the treatment of persistent oliguria or for tabe treatment of (SEQ ID NO:2) acute renal failure. It is preferred that the oligopeptide to be used in the production of the pharmaceutical composition consists of AQGV (SEQ ID NO:2).

Such pharmaceutical composition may be administered to the subject parenterally or orally. Such a pharmaceutical composition may consist essentially of oligopeptide and PBS. It is preferred that the oligopeptide is of synthetic origin. Suitable treatment for example entails administering the oligopeptide in the pharmaceutical composition to the patient intravenously in an amount of from about 0.25 to about 10 mg/kg body mass of the subject. It may be useful that the pharmaceutical composition consists essentially of from one to three different oligopeptides.

Such treatment is in particular preferred when the subject is undergoing persistent oliguria, for example when the subject's kidneys are not producing more than ½ ml urine per hour per kilogram body mass of the subject, or when the subject has a serum potassium level greater than 6.5 mmol per liter serum.

The thus developed chemical entity can be administered and introduced in-vivo systemically, topically, or locally. The peptide, or its modification or derivative, can be administered as the entity as such or as a pharmaceutically acceptable acid- or base-addition salt, formed by reaction with an inorganic acid (such as hydrochloric acid, hydrobromic acid, perchloric acid, nitric acid, thiocyanic acid, sulfuric acid, and phosphoric acid); or with an organic acid (such as formic acid, acetic acid, propionic acid, glycolic acid, lactic acid, pyruvic acid, oxatic acid, malonic acid, succinic acid, maleic acid, and fumaric acid); or by reaction with an inorganic base (such as sodium hydroxide, ammonium hydroxide, potassium hydroxide); or with an organic base (such as mono-, di-, trialkyl and aryl amines and substituted ethanolamines). A selected peptide and any of the derived entities may also be conjugated to sugars, lipids, other polypeptides, nucleic acids and PNA; and function in-situ as a conjugate or be released locally after reaching a targeted tissue or organ.

The compounds according to the general formula may be prepared in a manner conventional for such compounds. To that end, suitably N alpha protected (and side-chain protected if reactive side-chains are present) amino acid derivatives or peptides are activated and coupled to suitably carboxyl protected amino acid or peptide derivatives either in solution or on a solid support. Protection of the alpha-amino functions generally takes place by urethane functions such as the acid-labile tertiary-butyloxycarbonyl group ("Boc"), benzyloxycarbonyl ("Z") group and substituted analogs or the base-labile 9-fluoremyl-methyloxycarbonyl ("Fmoc") group. The Z group can also be removed by catalytic hydrogenation. Other suitable protecting groups include the Nps, Bmv, Bpoc, Aloc, MSC, etc. A good overview of amino protecting groups is given in The peptides, Analysis, Synthesis, Biology, Vol. 3 E. Gross and J. Meienhofer, eds. (Academic Press, New York, 1981). Protection of carboxyl groups can take place by ester formation, for example, base-labile esters like methyl or ethyl, acid labile esters like tert, butyl or, substituted, benzyl esters or hydrogenolytically. Protection of side-chain functions like those of lysine and glutamic or aspartic acid can take place using the aforementioned groups. Protection of thiol, and although not always required, of guanidino, alcohol and imidazole groups can take place using a variety of reagents such as those described in The Peptides, Analysis, Synthesis, Biology, id, or in Pure and Applied Chemistry, 59(3), 331-344 (1987). Activation of the carboxyl group of the suitably protected amino acids or peptides can take place by the azide, mixed anhydride, active ester, or carbodiimide method especially with the addition of catalytic and racemization-suppressing compounds like 1-N-N-hydroxybenzotriazole, N-hydroxysuccin-imide, 3-hydroxy-4-oxo-3,4 -dihydro-1,2,3-benzotriazine, N-hydroxy-5 norbornene-2,3-dicar-boxyimide. Also the anhydrides of phosphorus based acids can be used. See, e.g., The Peptides, Analysis, Synthesis, Biology, supra and Pure and Applied Chemistry, 59(3), 331-344 (1987).

It is also possible to prepare the compounds by the solid phase method of Merrifield. Different solid supports and different strategies are known see, e.g., Barany and Merrifield in The Peptides, Analysis, Synthesis, Biology, Vol. 2, E. Gross and J. Meienhofer, eds. (Acad. Press, New York, 1980), Kneib-Cordonier and Mullen Int. J. Peptide Protein Res., 30, 705-739 (1987) and Fields and Noble Int. J. Peptide Protein Res., 35, 161-214 (1990). The synthesis of compounds in which a peptide bond is replaced by an isostere, can, in general, be performed using the previously described protecting groups and activation procedures. Procedures to synthesize the modified isosteres are described in the literature e.g. for the --CH2--NH-- isostere and for the --CO--CH2 -- isostere.

Removal of the protecting groups, and, in the case of solid phase peptide synthesis, the cleavage from the solid support, can take place in different ways, depending on the nature of those protecting groups and the type of linker to the solid support. Usually deprotection takes place under acidic conditions and in the presence of scavengers. See, e.g., volumes 3, 5 and 9 of the series on The Peptides Analysis, Synthesis, Biology, supra.

Another possibility is the application of enzymes in synthesis of such compounds; for reviews see, e.g., H. D. Jakubke in The Peptides, Analysis, Synthesis, Biology, Vol. 9, S. Udenfriend and J. Mejenhofer, eds. (Acad, Press, New York, 1.987).

Although possibly not desirable from an economic point of view, oligopeptides according to the invention could also be made according to recombinant DNA methods. Such methods involve the preparation of the desired oligopeptide thereof by means of expressing recombinant polynucleotide sequence that codes for one or more of the oligopeptides in question in a suitable microorganism as host. Generally the process involves introducing into a cloning vehicle (e.g., a plasmid, phage DNA, or other DNA sequence able to replicate in a host cell) a DNA sequence coding for the particular oligopeptide or oligopeptides, introducing the cloning vehicle into a suitable eucaryotic or procaryotic host cell, and culturing the host cell thus transformed. When a eucaryotic host cell is used, the compound may include a glycoprotein portion.

A derivative or analogue can also be, for instance, generated by substitution of an L-amino acid residue with a D-amino acid residue. This substitution, leading to a peptide that does not naturally occur in nature, can improve a property of an amino acid sequence. It is, for example, useful to provide a peptide sequence of known activity of all D-amino acids in retro inversion format, thereby allowing for retained activity and increased half-life values. By generating many positional variants of an original amino acid sequence and screening for a specific activity, improved peptide derivatives comprising such D-amino acids can be designed with further improved characteristics.

A person skilled in the art is well able to generate analogous compounds of an amino acid sequence. This can, for instance, be done through screening of a peptide library. Such an analogue has essentially the same functional properties of the sequence in kind, not necessarily in amount. Also, peptides or analogues can be circularized, for example, by providing them with (terminal) cysteines, dimerized or multimerized, for example, by linkage to lysine or cysteine or other compounds with side-chains that allow linkage or multimerization, brought in tandem- or repeat-configuration, conjugated or otherwise linked to carriers known in the art, if only by a labile link that allows dissociation.

Synthetic versions of these oligopeptides as described above, and functional analogues or derivatives of these breakdown products, are herein provided to lower BUN concentration be used in methods to the treatment of disease.

The term "pharmaceutical composition" as used herein is intended to cover both the active composition of the invention alone or a composition containing the composition of the invention together with a pharmaceutically acceptable carrier, diluent or excipient. Acceptable diluents of an oligopeptide as described herein in the detailed description are for example physiological salt solutions or phosphate buffered salt solutions. In one embodiment, a signal molecule is administered in an effective concentration to an animal or human systemically, for example, by intravenous, intra-muscular or intraperitoneal administration. Another way of administration comprises perfusion of organs or tissue, be it in vivo or ex vivo, with a perfusion fluid comprising a signal molecule according to the invention. The administration may be done as a single dose, as a discontinuous sequence of various doses, or continuously for a period of time sufficient to permit substantial modulation of gene expression. In the case of a continuous administration, the duration of the administration may vary depending upon a number of factors that would readily be appreciated by those skilled in the art.

The administration dose of the active molecule may be varied over a fairly broad range. The concentrations of an active molecule that can be administered would be limited by efficacy at the lower end and the solubility of the compound at the upper end. The optimal dose or doses for a particular patient should and can be determined by the physician or medical specialist involved, taking into consideration well-known relevant factors such as the condition, weight and age of the patient, etc.

The active molecule may be administered directly in a suitable vehicle, such as, for example, phosphate-buffered saline ("PBS") or solutions in alcohol or DMSO. Pursuant to preferred embodiments of the present invention, however, the active molecule is administered through a single dose delivery using a drug-delivery system. A suitable drug-delivery system would be pharmacologically inactive or at least tolerable. It should preferably not be immunogenic nor cause inflammatory reactions, and should permit release of the active molecule so as to maintain effective levels thereof over the desired time period. Alternatives are known in the art as suitable for purposes of sustained release and are contemplated as within the scope of the present invention. Suitable delivery vehicles include, but are not limited to, the following: microcapsules or microspheres; liposomes and other lipid-based release systems; viscous instillates; absorbable and/or biodegradable mechanical barriers and implants; and polymeric delivery materials, such as polyethylene oxide/polypropylene oxide block copolymers, polyesters, cross-linked polyvinylalcohols, polyanhydrides, polymethacrylate and polymethacrylamide hydrogels, anionic carbohydrate polymers, etc. Useful delivery systems are well known in the art.

One formulation to achieve the active molecule release comprises injectable microcapsules or microspheres made from a biodegradable polymer, such as poly(dl-lactide), poly(dl-lactide-co-glycolide), polycaprolactone, polyglycolide, polylactic acid-co-glycolide, poly(hydroxybutyric acid), polyesters or polyacetals. Injectable systems comprising microcapsules or microspheres having a diameter of about 50 to about 500 micrometers offer advantages over other delivery systems. For example, they generally use less active molecules and may be administered by paramedical personnel. Moreover, such systems are inherently flexible in the design of the duration and rate of separate drug release by selection of microcapsule or microsphere size, drug loading and dosage administered. Further, they can be successfully sterilized by gamma irradiation.

The design, preparation, and use of microcapsules and microspheres are well within the reach of persons skilled in the art and detailed information concerning these points is available in the literature. Biodegradable polymers (such as lactide, glycolide and caprolactone polymers) may also be used in formulations other than microcapsules and microspheres; e.g., premade films and spray-on films of these polymers containing the active molecule would be suitable for use in accordance with the present invention. Fibers or filaments comprising the active molecule are also contemplated as within the scope of the present invention.

Another highly suitable formulation for a single-dose delivery of the active molecule in accordance with the present invention involves liposomes. The encapsulation of an active molecule in liposomes or multilamellar vesicles is a well-known technique for targeted drug delivery and prolonged drug residence. The preparation and use of drug-loaded liposomes is well within the reach of persons skilled in the art and well documented in the literature.

Yet another suitable approach for single-dose delivery of an active molecule in accordance with the present invention involves the use of viscous installates. In this technique, high molecular weight carriers are used in admixture with the active molecule, giving rise to a structure that produces a solution with high viscosity. Suitable high molecular weight carriers include, but are not limited to, the following: dextrans and cyclodextrans; hydrogels; (cross-linked) viscous materials, including (cross-linked) viscoelastics; carboxymethylcellulose; hyaluronic acid; and chondroitin sulfate. The preparation and use of drug-loaded viscous instillates is well known to persons skilled in the art.

Pursuant to yet another approach, the active molecule may be administered in combination with absorbable mechanical barriers such as oxidized regenerated cellulose. The active molecule may be covalently or non-covalently (e.g., ionically) bound to such a barrier, or it may simply be dispersed therein.

The invention is further explained with the aid of the following illustrative examples.

### EXAMPLES

Six oligopeptides (i.e., A: {LAGV (SEQ ID NO:4)}, B: {AQGV (SEQ ID NO:2)}, C: {LAG}, D: {AQG}, E: {MTR}, and F: {MTRV (SEQ ID NO:1)}) peptides A,C, D and E not being part of the invention as claimed were tested and compared with PBS (control) in a double blind animal study for each peptide's relative ability to aid recovery in a mouse renal ischemia reperfusion test. In this test, the mice were anesthetized, and one kidney from each mouse was removed. The other kidney was tied off for 25 minutes, and the serum urea levels were allowed to increase. Both before and after tying off, each of the separate peptides was administered to thirty (30) different mice (5 mg oligopeptide / kg body mass intravenously), after which, the mortality of the mice was determined for each oligopeptide as well as was the BUN concentration at two hours, 24 hours and 72 hours. The results are shown in FIG. 1 and below.

Under inhalation anaesthesia, the left kidney with its artery and vein was isolated and occluded for 25 minutes using a microvascular clamp. During surgery animals were placed on a heating path to maintain body temperature at 37oC. Five minutes before placing the clamp, and 5 minutes before releasing the clamp, 5 mg/kg of peptide, dissolved in 0.1 mL of sterile saline, was administered intravenously. After reperfusion of the left kidney the right kidney was removed. Kidney function was assessed by measuring blood urea nitrogen before clamping, and at 2, 24, and 72 hours after reperfusion.

### Results

Mortality at 72 hours postreperfusion.

| PBS | A (LAGV) | B (AQGV) | C (LAG) | D (AQG) | E (MTR) | F (MTRV) |
|---|---|---|---|---|---|---|
| 6/10 | 6/10 | 0/10 | 4/10 | 4/10 | 4/10 | 2/10 |
| *P< (vs PBS) | NS | 0.01 | 0.01 | 0.01 | 0.01 | 0.01 |

| | | | | | | |
|---|---|---|---|---|---|---|
| *2x2 Chi-square test, df=1 | | | | | | |

Peptide A was the first peptide administered in the renal ischemia reperfusion test. The personnel who performed the experiments went through a learning curve while working with peptide A. During administration of the peptide in the inferior caval vein, some animals experienced moderate blood loss from the site of injection, whereas others did not. Inadvertently the animals were returned to the stable without drinking water present in their cages the first night after surgery. Also, by mistake, the animals that were intended to be sacrificed at 72h were killed 48h after reperfusion. None of these or other problems were encountered during the experiments with peptides B-F. Taken together we find that the results obtained with Peptide A may not be not reliable, and peptide A should be tested again.

As can be seen, mice administered the oligopeptides MTRV (SEQ ID NO:1) and especially AQGV (SEQ ID NO:2) did much better in terms of both survival (a significant reduction in mortality versus the PBS control group) and reduced BUN concentration than the control group (PBS) or the group administered the other oligopeptides, with more mice surviving and the serum urea levels being much lower than in the other groups. However, the oligopeptides LAG, AQG, and MTR, having no effect on BUN concentration, each caused a significant reduction of mortality compared to the PBS control.

## Claims

1. Use of an oligopeptide having the sequence MTRV (SEQ ID NO:1) or AQGV (SEQ ID NO:2), or an acceptable salt thereof, for the manufacture of a medicament for the treatment of persistent oliguria.

2. Use of an oligopetide having the sequence MTRV (SEQ ID NO:1) or AQGV (SEQ ID NO:2), or an acceptable salt thereof, for the manufacture of a medicament for the treatment of acute renal failure.

3. Use according to claim 1 or 2, wherein the oligopeptide consists of AQGV (SEQ ID NO:2).

4. Use according to any one of claims 1-3, wherein the medicament is formulated to be administered to a subject parenterally.

5. Use according to any one of claims 1-3, wherein the medicament is formulated to be administered to a subject orally.

6. Use according to any one of the preceding claims, wherein the medicament consists essentially of oligopeptide and PBS.

7. Use according to any one of the preceding claims, wherein the oligopeptide is of synthetic origin.

8. Use according to any one of the preceding claims, wherein the oligopeptide is to be administered intravenously in an amount of about 0.25 to about 10 mg/kg body mass of a subject.

9. Use according to any one of the preceding claims, wherein persistent oliguria or acute renal failure is **characterized by** a serum potassium level greater than 6.5 mmol per liter serum.

10. Use according to any one of the preceding claims, wherein persistent oliguria or acute renal failure is **characterized by** the production of not more than 0.5 ml urine per hour per kilogram body mass of a subject.

## Patentansprüche

1. Verwendung eines Oligopeptids mit der Sequenz MTRV (SEQ ID NO: 1) oder AQGV (SEQ ID NO: 2) oder ein akzeptables Salz derselben, für die Herstellung eines Medikamentes zur Behandlung der persistierenden Oligurie.

2. Verwendung eines Oligopeptids mit der Sequenz MTRV (SEQ ID NO: 1) oder AQGV (SEQ ID NO: 2) oder eines akzeptablen Salzes derselben, für die Herstellung eines Medikamentes zur Behandlung des akuten Nierenversagens.

3. Verwendung nach Anspruch 1 oder 2, wobei das Oligopeptid aus AQGV (SEQ ID NO: 2) besteht.

4. Verwendung nach einem der Ansprüche 1 bis 3, wobei das Medikament formuliert ist, um parenteral an ein Individuum verabreicht zu werden.

5. Verwendung nach einem der Ansprüche 1 bis 3, wobei das Medikament formuliert ist, um oral an ein Individuum verabreicht zu werden.

6. Verwendung nach einem der vorhergehenden Ansprüche, wobei das Medikament im Wesentlichen aus Oligopeptid und PBS besteht.

7. Verwendung nach einem der vorhergehenden Ansprüche, wobei das Oligopeptid synthetischen Ursprungs ist.

8. Verwendung nach einem der vorhergehenden Ansprüche, wobei das Oligopeptid intravenös in einer Menge von etwa 0,25 bis etwa 10 mg/kg Körpermasse eines Individuums zu verabreichen ist.

9. Verwendung nach einem der vorhergehenden Ansprüche, wobei die persistierende Oligurie oder das akute Nierenversagen durch einen Serum-Kalium-Spiegel von größer als 6,5 mmol/l Serum **gekennzeichnet** ist.

10. Verwendung nach einem der vorhergehenden Ansprüche, wobei die persistierende Oligurie oder das akute Nierenversagen durch die Produktion von nicht mehr als 0,5 ml Urin pro Stunde pro Kilogramm Körpermasse eines Subjektes **gekennzeichnet** ist.

## Revendications

1. Utilisation d'un oligopeptide présentant la séquence MTRV (SEQ ID NO:1) ou AQGV (SEQ ID NO:2), ou un sel en dérivant, pour la fabrication d'un médicament pour le traitement d'une oligurie persistante.

2. Utilisation d'un oligopeptide présentant la séquence MTRV (SEQ ID NO:1) ou AQGV (SEQ ID NO:2), ou un sel en dérivant, pour la fabrication d'un médicament pour le traitement d'une insuffisance rénale aiguë.

3. Utilisation selon la revendication 1 ou. 2, dans laquelle l'oligopeptide consiste en AQGV (SEQ ID NO:2).

4. Utilisation selon une quelconque des revendications 1 à 3. dans laquelle le médicament est mis sous une forme destinée à être administrée par voie parentérale à un patient.

5. Utilisation selon une quelconque des revendications 1 à 3. dans laquelle le médicament est mis sous une forme destinée à être administrée par voie orale à un patient.

6. Utilisation selon une quelconque des revendications précédentes, dans laquelle le médicament consiste essentiellement en oligopeptide et PBS.

7. Utilisation selon une quelconque des revendications précédentes, dans laquelle l'oligopeptide est d'origine synthétique.

8. Utilisation selon une quelconque des revendications précédentes, dans laquelle l'oligopeptide est destiné à être administré par voie intraveineuse en une quantité d'environ 0,25 à environ 10 mg/kg de masse corporelle du patient..

9. Utilisation selon une quelconque des revendications précédentes, dans laquelle l'oligurie persistante ou l'insuffisance rénale aiguë est **caractérisée par** un taux de potassium dans le sérum supérieur à 6,5 mmol par litre de sérum.

10. Utilisation selon une quelconque des revendications précédente, dans laquelle l'oligurie persistante ou l'insuffisance rénale aiguë est **caractérisée par** la production de pas plus de 0,5 ml d'urine par heure par kilogramme de masse corporelle du patient.
